# EUROPEAN PATENT APPLICATION

(11) **EP 0 907 079 A1**
(43) Date of publication of application: **07.04.1999**
(21) Application number: 98116681.2
(22) Date of filing: 03.09.1998
(51) Int. Cl.: G01N 33/36, D02J 1/08

(54) **Quality control apparatus for textured filament yarn**

(30) Priority: 02.10.1997 JP 269769/97
(71) Applicant: Murata Kikai Kabushiki Kaisha, Minami-ku, Kyoto-shi, Kyoto 601 (JP)
(72) Inventor: Nakade, Kazuhiko, Ukyo-ku, Kyoto-shi (JP)
(74) Representative: Liedl, Christine, Dipl.-Chem.

(57) **Abstract**

To provide a quality control apparatus for a texturing filament yarn capable of directly assessing the quality of the textured filament yarn formed by applying a given texture processing to one or multiple filament yarns, a yarn supervising means 5 for detecting the external shape of a textured filament yarn Y3 is provided between feed rollers 2 and 3 at which the textured filament yarn Y3 is maintained in an overfeed state, that is, at the exit of a heater 4. In addition, in an evaluation means 6 for evaluating yarn quality from its external shape, the unevenness degree U% of yarn is employed as an indicator for evaluating yarn quality.

## Description

### Field of the Invention

The present invention relates to a quality control apparatus for a textured filament yarn formed by applying a given texture processing to one or multiple filament yarns (i.e., an aggregation of filaments, hereinafter referred to as " filament yarn").

### Background of the Invention

Several types of machines including air processing machine, draw texturing machines, etc. are available for manufacturing a textured filament yarn by applying texture processing to a filament yarn comprising of a number of filaments. An air processing machine also serves as a yarn doubling machine, in which a plurality of filament yarns such as POY (Partial Oriented Yarn) or FOY (Full Oriented Yarn) or the like are doubled, and an interlace (entangling) is generated between a plurality of filament yarns due to air, or one of two filament yarns is employed as a core yarn and the other is employed as a sheath yarn, and a sheath yarn is entangled around the core yarn by air action. Such a texture processing is applied and, as required, heat treatment is done by means of a primary heater or a secondary heater which is used either before or after the texture processing.

A draw texturing machine, on the other hand, performs a twisting and untwisting by nipping the yarn at a crossing portion of two running belts to which a contact pressure is applied while one filament yarn is drawing, and the yarn is then fixed in a twisted state by heat treatment.

In a air processing machine, a yarn cut detecting sensor is arranged with respect to each of a plurality of filament yarns before texture processing in order to detect single yarn cuts (i.e., a cut of one of the filament yarns). Conventionally, the quality of textured filament yarn itself has not been evaluated automatically, instead, the finished winding package has been checked visually by a worker skilled in this art. However, this approach has a problem, because air-based texture processing methods are often assessed for failure during a dying process, hence the finished winding package cannot be checked visually by an unskilled worker.

In a draw texturing machine, a untwisting side tension T2 is detected by means of a tension sensor provided downstream of a twister using a running belt, and the quality of the textured filament yarn is evaluated by using abnormal values of this untwisting side tension T2. However, this evaluation has not been directed to the textured filament yarn itself and has instead been performed only indirectly.

The present invention has been developed to address problems inherent in the prior art. It is intended to provide a quality control apparatus for a textured filament yarn capable of directly checking the quality of textured filament yarns formed by applying a given texture processing to one or multiple filament yarns.

### Summary of the Invention

The present invention as claimed in claim 1 is directed to a quality control apparatus for a textured filament yarn comprising a texture processing means for applying a given texture processing to one or multiple filament yarns in order to form a textured filament yarn, a yarn supervising means for detecting the external shape of the textured filament yarn in a non-contact state and an evaluating means for evaluating yarn quality from the external shape, and the yarn supervising means is provided at a area where the filament yarn is maintained in an overfeed state.

When the textured filament yarn is placed in the overfeed state, its external shape is changed significantly due to the influence of the texture processing means. Here, the texture processing means may be interlace processing, tasran processing, draw texturing processing, or any other processing in which the yarn's external shape is changed as a result of processing. An air processing machine doubles a plurality of filament yarns and applies interface processing or tasran processing. A draw texturing machine applies draw processing and false-twist processing to a single filament yarn, and as required, further applies interlace processing. A textured filament yarn is manufactured using the air processing machine or draw texturing machine. To evaluate yarn quality, one useful indicator is the unevenness degree of yarn (i.e., the CV or U value) which evaluates differences produced by interlace or tasran processing. Other indicators are available for nep, thick portions, or thin portions, as determined by segmenting the thickness or length of swelling portions in the yarn, and for short-period unevenness, long-period unevenness and related matters.

The present invention as claimed in claim 2 is directed to a quality control apparatus for a textured filament yarn comprising: a texture processing means for applying a given texture processing to one or multiple filament yarns to form a textured filament yarn, a yarn supervising means for detecting the external shape of the textured filament yarn in a non-contact state and an evaluating means for evaluating a yarn quality from the external shape, where the evaluating means calculates the unevenness degree of yarn as an indicator for evaluating yarn quality.

When the unevenness degree of yarn is employed as an indicator, it yields more significant differences than other indicators. That is, while indicators for nep, thick places or thin places, as determined by segmenting thickness and length of a swelling portion, or indicators for short-period unevenness or long-period unevenness can also be used, when the unevenness degree of yarn (CV value or U value) is employed, it causes significant differences in interlace or tasran processing.

The present invention as claimed in claim 3 is directed to a photoelectric detector, wherein the yarn supervising means comprises a light projecting element and a light receiving element.

The present invention as claimed in claim 4 is characterized in that the textured filament processing yarn is formed by applying fluid entangling processing to one or multiple filament yarns, using the configuration outlined in any of claims 1 to 3.

The present invention as claimed in claim 5 is characterized in that the textured filament yarn is formed by entangling a sheath yarn to a core yarn, using the configuration outlined in any of claims 1 to 3.

### Brief Description of the Drawing

Figure 1 is a constructive view of important portions of a quality control apparatus for a textured filament yarn according to the present invention.
Figure 2 is a view showing a process for a external shape signal.
Figure 3 is a configuration view of important portions of another quality control apparatus for a textured filament yarn according to the present invention.
Figure 4 is a view showing a process for an external shape signal.
Figure 5 is a wiring diagram showing an arrangement of a quality control apparatus for a textured filament yarn according to the present invention.
Figure 6 is a view of equipment configuration for an air processing machine.
Figure 7 is a view showing a textured filament yarn produced by the air processing machine.
Figure 8 is a view of equipment configuration for a draw texturing machine.
Figure 9 is a view showing a textured filament yarn produced by the draw texturing machine.
Figure 10 is a graph depicting an output example of a yarn supervising means for interlace yarn.
Figure 11 is a view showing an example of an alarm indicated by a quality control apparatus according to the present invention.

### Detailed Description of the Preferred Embodiments

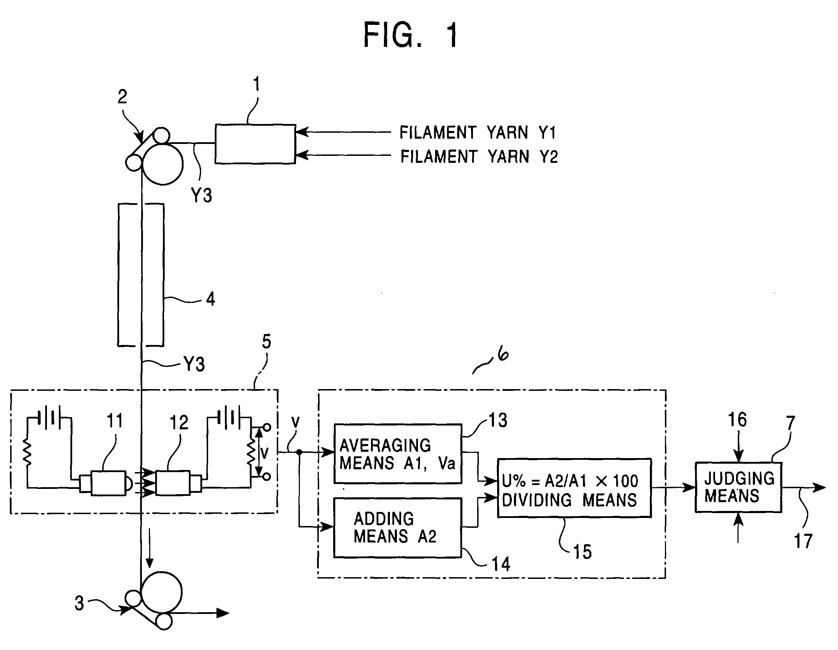

Below, preferred embodiments of the present invention are described with reference to the accompanying drawings. Figure 1 is a constructive view of important elements of a quality control apparatus for a textured filament yarn according the present invention.

In Figure 1, 1 is a texture processing means for applying texture processing such as interlacing by doubling a filament yarn Y1 and a filament yarn Y2 through an air ejecting nozzle, 2 and 3 are upstream side and downstream side feed rollers for feeding a textured filament yarn Y3 to which texture processing is applied in an overfeed state, 4 is a secondary heater employed as required, 5 is a yarn supervising means, 6 is a calculation means and 7 is a judging means.

The yarn supervising device 5 comprises a light emitting diode (light projecting element) 11 and a photo-transistor (light receiving element) 12. This device detects the quantity of light projected from the light emitting diode 11 by means of the photo-transistor 12, outputs the detected light quantity as an electric variation (voltage) V, and has high sensitivity and responsiveness using external shape detection methods. In particular, in the case of a filament yarn, since the number of filaments is identical, it cannot be detected by using an electrostatic capacitance method. Therefore, the yarn supervising device 5 using a photo-electric detector comprising the light emitting diode 11 and the photo-transistor 12 is employed.

The calculation means 6 comprises an averaging means 13, an adding means 14 and a dividing means 15. The averaging means 13 moves and adds a detection signal (external shape) V from the yarn supervising device 5 to calculate an addition value A1 and an average value Va of the detection signal V. The adding means 14 moves and adds the difference between the value of the detection signal V from the yarn supervising device 5 and the average value Va of the external shape to calculate a deviation addition value A2. The dividing means 15 divides this deviation addition value A2 by the addition value A1 of the external shape, and calculates a unevenness degree U% of yarn. The judging means 7 compares the unevenness degree U% of yarn with a yarn-unevenness-degree signal 16 from the other texture processing unit (not shown in the drawing), and outputs normal and abnormal signals 17. In specific terms, for example, an average value of the unevenness degree of yarn of the given number of processing units or all processing units is calculated, and it is judged to be abnormal when the unevenness degree of yarn of the processing unit being judged deviates over a given value from the average value.

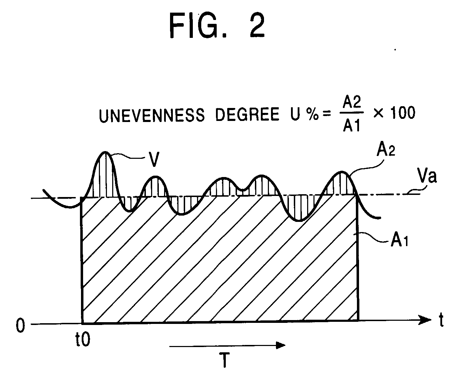

Figure 2 is a view showing a process for the external shape signal of this yarn. When the value of an external shape V is moved and added in a certain zone T, a moving additional value A1 can be obtained, and a moving average value Va can be calculated by dividing this value A1 by the number of data. The difference between the value of the external shape signal V and the external shape average value Va is moved and added, to calculate a deviation addition value A2. This deviation addition value A2 is divided by the external shape addition value A1 and multiplied by 100, thus yielding the unevenness degree U% of yarn. Since values obtained by dividing the deviation addition value A2 by the number of data represent the average deviation of the external shape, this unevenness degree U% of yarn is obtained by expressing the average deviation of the external shape in percentage terms with respect to the average value Va of the external shape.

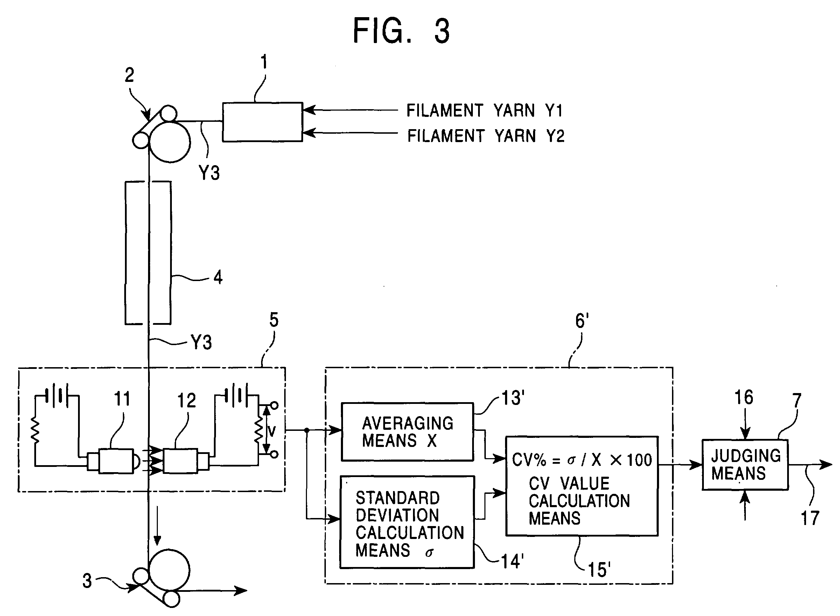

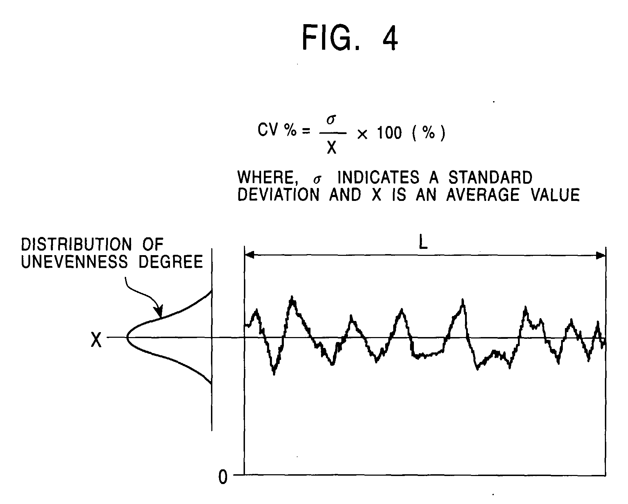

Figures 3 and 4 show examples when a CV value is employed as the unevenness degree of yarn, instead of a U value. In Figure 3, a portion different from that in Figure 1 is configured by calculation means 6'. The calculation means 6' comprises an averaging means 13', a standard deviation calculation means 14' and a CV value calculation means 15'.

In Figure 4, the averaging means 13' calculates an average value x, the standard deviation calculation means 14' calculates a standard deviation σ and the CV value calculation means 15' calculates a CV value (%) as ( σ/x) × 100. Both the U and CV values are indicators representing the unevenness degree of yarn. Even if only one is employed for evaluation, there should be no substantial difference in evaluation results.

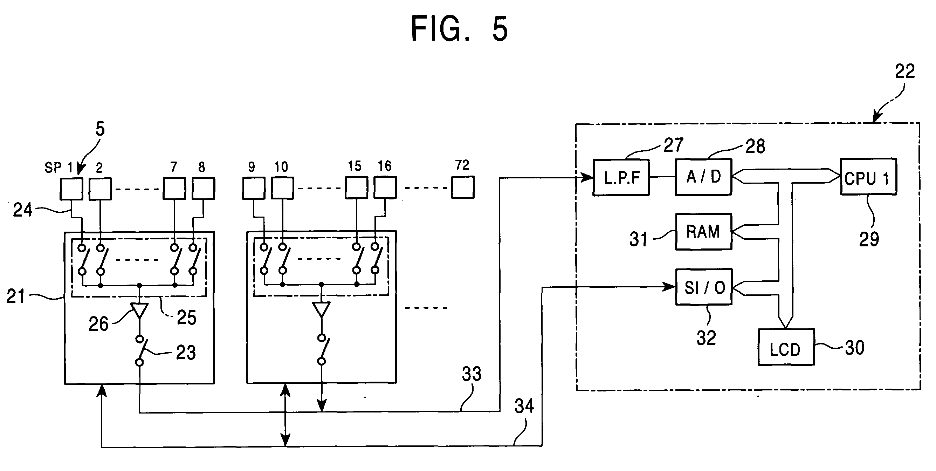

Figure 5 is a wiring diagram of the quality control apparatus when a number of texture processing units are provided in array.

21 is a slave provided every span of 12 units and 22 is an analytical device master installed in common to a plurality of slaves 43. Analysis of each slave 21 is performed by a sequentially switching switch means 23. An electric signal 24 from the yarn supervising device 5 installed on each unit U belonging to the slave 21 is transmitted to a multiplexer 25, sequentially switched and fetched, amplified by means of an analyzer 26, and transmitted to a master 22 via a wire 33. The electric signal transmitted to the master 22 is input to an A/D converter 28 via a low-pass filter 27, digitized there, and input to a master computer 29.

The calculation means 6 and the judging means 7 described in Figure 1 are stored as a program in the master computer 29. This master computer 29 analyzes these electric signals. If an abnomality is found, an indicator 30 such as an LCD display the abnormality. If required, a faulty unit is stopped via commnication lines (not shown in the drawing). In addition, information on abnormal states is stored in a storage means 31 so as to be available for analysis of individual units. An input/output port 32 has a wire 34 connected to sequentially switch the slave 21.

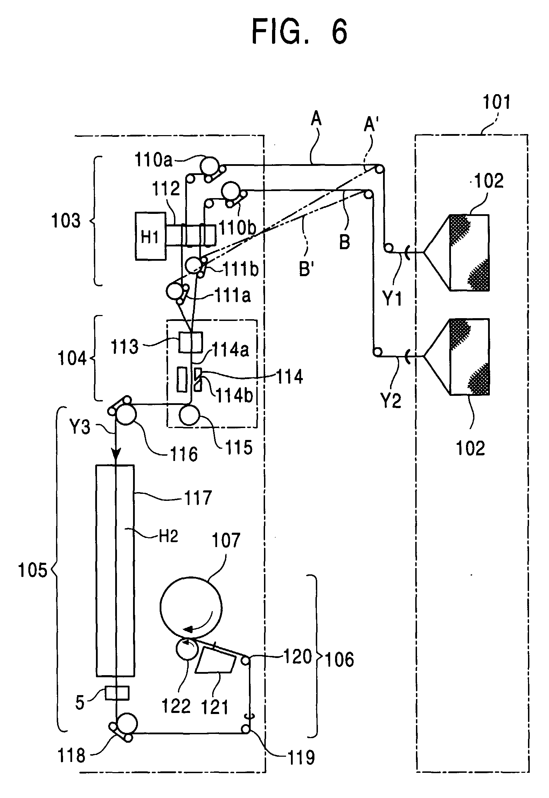

Below, we explain an example of an air processing machine to which the quality control apparatus associated with the present invention by using Figure 6. Figure 6 shows one unit of the equipment arrangement. One unit of the air processing machine sequentially comprises a creel stand 101, a draw texturing processing portion 103, a texture processing portion 104, a thermal treatment portion 105, and a winding portion 106 in array form. For the creel stand 101, two kinds of yarn supply packages 102 are supported, and filament yarns Y1 and Y2 are repeated at the draw texturing portion 103 in two paths, i.e., in lines A and B.

The draw texturing portion 103 is provided with two pairs of upstream side feed rollers 110a, 110b and two pairs of downstream side feed rollers 111a, 111b, with respect to a heating pin 112 serving as a primary heater, so that texturing processing is selectively applied to two filament yarns Y1, Y2 in lines A and B, line A' only, or line B' only. The filament yarns Y1, Y2, having been wound around the heating pin 112 several times, are heated to drawing temperature by the heating pin 112 and drawn between the upstream side feed rollers 110a, 110b and the downstream side feed rollers 111a, 111b, to achieve the desired drawing performance. In general, drawing is performed for POY (Partial Oriented Yarn) but not for FOY (Full Oriented Yarn). This makes mixed textures of POY+FOY possible.

A texture processing portion 104 comprises a water imparting device 113, an ejecting nozzle 114 and a shock material 115. The water imparting device 113 imparts water to the yarns Y1, Y2 by either the water-bath or water-guide methods. The water imparting device 113 increases the nominal mass of the yarns Y1, Y2, thus enhancing the efficiency of opening and entangling action by the ejecting nozzle 114 and the shock material 115. It is possible that one of the yarns Y1, Y2 can pass through the water imparting device 113, while the other cannot. The ejecting nozzle 114 is structured so that a compressed air path 114b is opened diagonally to a yarn path 114a, and the yarns Y1, Y2 are opened by compressed air ejection. As a shock material 115, a baffle bar, a baffle plate, or a baffle ball is provided at the exit of the ejecting nozzle 114. By allowing the yarns Y1, Y2 to be ejected from the ejecting nozzle 114 to collide and bend, turbulence is created by compressed air and filaments becoming randomly bent by each other, thus forming a loop.

The heat treatment portion 105 comprises an upstream side feed roller 116, a secondary heater 117 and a downstream side feed roller 118. A filament processing yarn Y3 is adequately slackened between the upstream side feed roller 116 and the downstream side feed roller 118 while a loop or the like is thermally set by means of the secondary heater 117. For this thermal setting, the surface speed of the upstream side feed roller 116 is kept slightly slower than that of the downstream side feed roller 118, namely, overfed, and the filament processing yarn Y3 is fed out. That is, the filament processing yarn Y3 is kept slightly slackened, yielding the greatest external variation in the yarn due to texture processing. The yarn supervising means 5 is provided at the exit of the secondary heater 117, that is, between the secondary heater 117 and the downstream side feed roller 118.

The winding portion 106 comprises a yarn guide 119, a yarn guide bar 120, a traverse apparatus 121 and a friction roller 122. The textured filament yarn Y3 delivered from the downstream side feed roller 118 is traversed by means of the traverse apparatus 121 via the yarn guide 119 and the yarn guide bar 120.

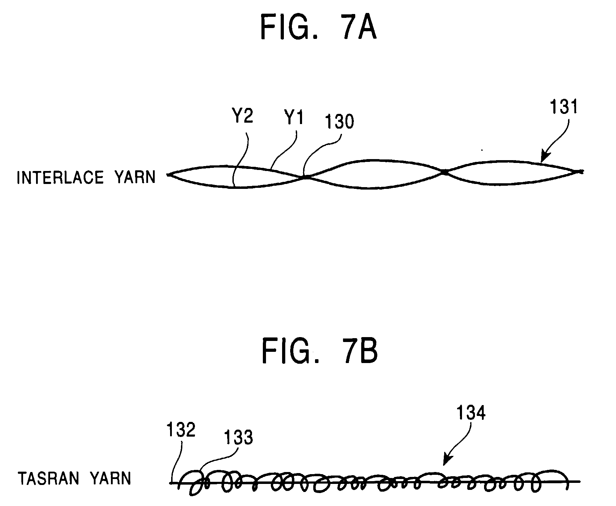

In such an air processing machine, various texture processing can be performed once an adequate processing means is arranged at the texture processing portion 104. As shown in Figure 7A, an interlace yarn 131 can be processed such that an interlace portion 130 is formed using given intervals of the two yarns Y1, Y2. In addition, as shown in Figure 7B, when one yarn is employed as a core yarn 132 and the other as a sheath yarn 133, a core-sheath yarn 134 can be processed in which the sheath yarn 113 is lapped around the core yarn 132. Changes in the external shape of interlace yarn 131 are minimal when tension is applied. However, in an overfeed state, the yarn's external shape changes significantly, as shown in Figure 7A. Further, external shape of the core-sheath yarn 134 changes in normal state. However, in the overfeed state, these changes in external shape are clearer and more visible.

Even during draw texturing processing of a single filament yarn, texture processing is applied for reasons given below. Since a single filament yarn having no twist lacks cohesion property, when used as a warp in an weaving machine, a knitting machine, or the like adopted for post-processing, the individual filaments constituting filament yarn become separated from each other, resulting in yarn cut, intertwining, or woven fabric failures, and the single filament yarn having no twist is difficult to use. Therefore an entanglement portion is partly formed by applying fluid ejection to the draw textured yarn so as to impart cohesion property to the filament yarn.

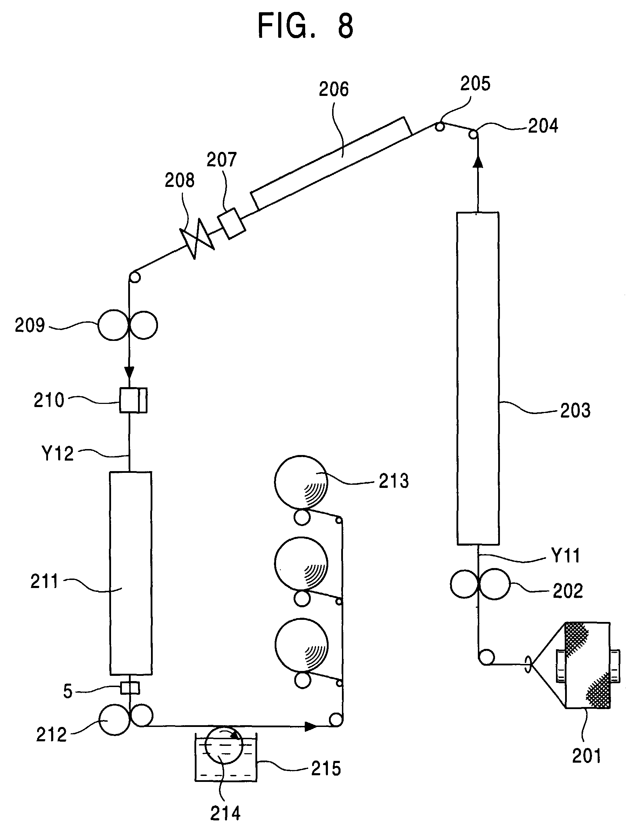

Figure 8 shows an example of such a draw texturing machine, to which a quality control apparatus of the present invention is applied.

A yarn Y11 supplied from a yarn supply package 201 by means of a first feed roller 202 raises through a primary heater 203 and is cooled to a set temperature through a balloon control plate 206 and a cooling device 207 via alignment rollers 204, 205. Thereafter, the yarn Y11 is introduced into a publicly known false-twisting device 208 having a belt-based or similar design, is subjected to temporary twisting action, and is then drawn and textured. Further, the yarn is actively fed outward by means of a second feed roller (upstream side feed roller) 209 and is introduced into a fluid ejecting nozzle 210. A yarn Y12 to which cohesion property is imparted by means of fluid ejection may be fed at by means of a third feed roller (downstream side feed roller) 212 through a secondary heater 211, and oil is supplied to the yarn Y12 by means of an oiling roller 214 which is a type of oiling device, and then the yarn Y12 is wound on a winding package 213. This oiling roller 214 rotates at low speed and its roller 214 is soaked in an oil bath 215. Oil adhering to the surface of the oiling roller 214 is supplied to the yarn as it moves across the surface of the oiling roller 214.

When the secondary heater 211 is to be used, the surface speed of the second feed roller 209 is set to produce overfeed, namely, to be slightly slower than the surface speed of a third feed roller 212. The yarn supervising means 5 is provided at the exit of the secondary heater 211, which corresponds to this overfeed zone, more specifically, it is between the secondary heater 211 and the third feed roller 212. That is, the yarn supervising means 5 is provided at a position at which a textured filament yarn is slightly slackened and the external variations induced by texture processing are most significant.

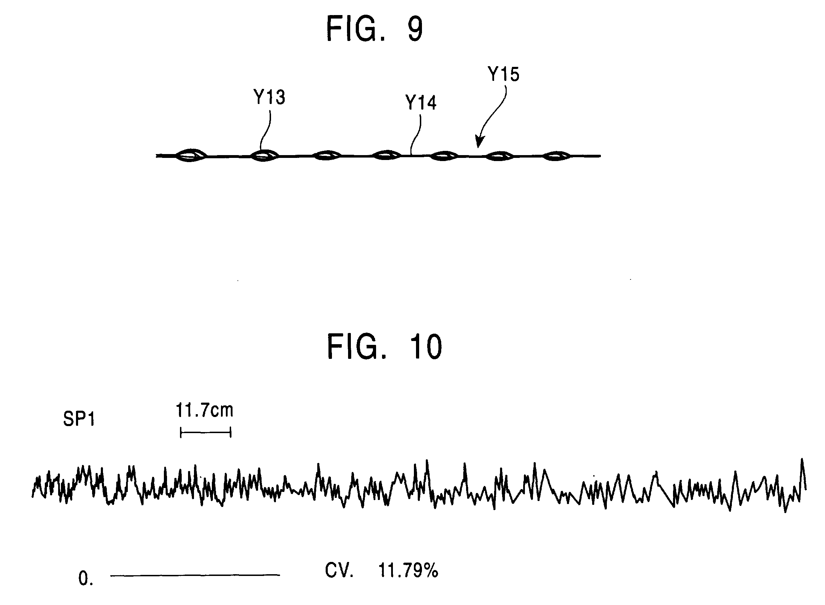

The yarn Y thus obtained is a bulky yarn Y15 which is textured and crimped as a whole, as shown schematically in Figure 9. A thick yarn portion Y13 and an interlace portion Y14 appear alternately, and at the interlace portion Y14, single filaments constituting filament bundles are interlaced with each other by fluid action, intertwine, and are provided with cohesion properties by means of loops or the like. When such a textured filament yarn is overfed, an external shape due to the thick yarn portion Y13 and the interlace portion Y14 is clearly produced. Thus, since the thick yarn portion Y13 and the interlace portion Y14 appear alternately, it is possible to evaluate an indicator for nep, thinning, and thickness.

An air processing machine and a draw texturing machine are exemplified as having texture processing means. However, without any limitation thereto, a fluid ejecting nozzle is provided with respect to upstream side yarn in the take up winder for a filament yarn, thereby making it possible to apply the present invention.

As shown in Figure 7A, Figure 10 indicates an output V in which a change in the external shape of an interlace yarn is detected by a photoelectric yarn supervising means located in an overfeed zone. Each peak is an interlace portion, and about 100/m interlace portions exist. The CV value shown is 11.79.

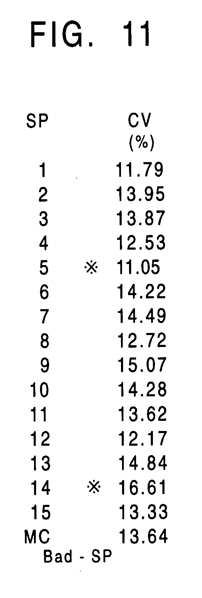

In an example, the CV value of each unit SP1 to 15 is represented in Figure 11. The average CV value of all units SP1 to 15 is 13.64%, the minimum value is 11.05% (SP5) and the maximum value, 16.61% (SP14). The standard deviation of the CV value of each unit SP1 to 11 is 1.46%, the 3σ-value is equal to 4.37%. Since the CV value for different units vary for general spun yarn by 1 to 2%, the difference between units for interlace yarn appears to be at least twice as great. When texture processing is applied to a filament yarn, it is not expected conventionally that such a deviation is produced.

In Figure 11, units SP5 and SP14, which have great deviation, are starred, these units are found to have different tendencies than other units. Thus, when the CV values of these two units are compared with those of other units, potentially faulty units can be located.

According to the present invention as claimed in claim 1, a yarn supervising means is provided at an area where the yarn is maintained in an overfeed state, such that the quality of textured filament yarn can be precisely assessed. In particular, since unevenness in the filament yarn is eliminated in a drawing portion, the differences in the external shape of the textured filament yarn based on the texturing processing exhibit obviously by means of the yarn supervising means provided at an area where the yarn is maintained in an overfeed state, thus enabling yarn quality to be accurately assessed. That is, the textured filament yarn produced by means of an air processing machine which has been visually checked in the conventional method can be continuously evaluated in the present invention. In addition, the textured filament yarn produced by means of a draw texturing machine has been conventionally evaluated indirectly, based on untwisting tension change, however, the yarn can be directly evaluated, based on changes in its external shape in the present invention.

According to the present invention as claimed in claim 2, since the unevenness degree of yarn is used as an indicator for evaluating yarn quality, it can be used to indicate whether units with specific texturing processing means have mechanical problems compared with the other units, without any limitation on the precise evaluation of the textured filament yarn's quality. This feature makes it easier to maintain air processing and draw texturing machines, and enhances operating performance.

According to the present invention as claimed in claim 3, in the case of a filament yarn, since the number of filaments is identical, it cannot be precisely detected by using an electrostatic capacitance method. Using a photoelectric method, even if the number of filaments is identical, as long as differences in the external shape are produced, thereby making it possible to precisely evaluate a yarn quality.

According to the invention as claimed in claim 4, it is possible to evaluate interlace yarn quantity by evaluating such forms of yarn failure as those caused by the fluid ejecting nozzle. Thereby, it enables operators to judge whether the fluid ejecting nozzle is functioning properly, and can also be employed for maintenance information, such as replacement requirements.

According to the invention as claimed in claim 5, it is possible to evaluate the quality of core and sheath yarns by assessing such forms of yarn failure as those caused by the fluid ejecting nozzle. Thereby, it enables operators to judge whether the texture processing means is functioning properly for tasran processing, and can also be employed for maintenance information, such as replacement requirements.

## Claims

1. A quality control apparatus for a textured filament yarn comprising:
a texture processing means for applying texture processing to one or multiple filament yarns to form a textured filament yarn;
a yarn supervising means for detecting the external shape of said textured filament yarn while in a non-contact state;
and an evaluating means for evaluating yarn quality from said external shape, with said yarn supervising means being provided in the area through which filament yarn runs while in an overfeed state.

2. A quality control apparatus for a textured filament yarn comprising:
a texture processing means for applying texture processing to one or multiple filament yarns to form a textured filament yarn;
a yarn supervising means for detecting the external shape of said filament processing yarn in non-contact state; and
an evaluating means for evaluating a yarn quality from said external shape, with said evaluating means calculating unevenness degree of yarn as an indicator for evaluating yarn quality.

3. A quality control apparatus for a textured filament yarn as in claim 1 or claim 2, wherein said yarn supervising means is a photoelectric detector provided with light projecting and receiving elements.

4. A quality control apparatus for a textured filament yarn as in any one of claims 1 to 3, wherein said textured filament yarn is formed by applying fluid entangling processing to one or multiple filament yarns.

5. A quality control apparatus for a textured filament yarn as in any one of claims 1 to 3, wherein said textured filament yarn is formed by entangling a sheath yarn with respect to a core yarn.
